(19) Europäisches Patentamt
European Patent Office
Office européen des brevets

(11) Veröffentlichungsnummer : **0 146 889**
**B1**

(12) **EUROPÄISCHE PATENTSCHRIFT**

(45) Veröffentlichungstag der Patentschrift : **18.06.86**

(21) Anmeldenummer : **84115407.3**

(22) Anmeldetag : **14.12.84**

(51) Int. Cl.⁴ : **C 07 C143/58**, C 07 C143/56, C 07 C139/00

# (54) Verfahren zur Herstellung von aromatischen Aminosulfonsäuren.

(30) Priorität : **29.12.83 DE 3347452**

(43) Veröffentlichungstag der Anmeldung : **03.07.85 Patentblatt 85/27**

(45) Bekanntmachung des Hinweises auf die Patenterteilung : **18.06.86 Patentblatt 86/25**

(84) Benannte Vertragsstaaten : **BE CH DE FR GB LI NL**

(56) Entgegenhaltungen :
**EP-A- 0 000 634**
**CH-A- 632 990**
**DE-A- 2 240 849**
**DE-A- 2 301 739**
**DE-A- 3 003 254**
**DE-B- 2 362 781**
**DE-B- 2 538 307**
**CHEMICAL ABSTRACTS, Band 78, Nr. 5, Februar 1973, Columbus, Ohio, USA SHMONINA, V.P. et al. "Development of a catalytic system based on Raney-nickel for reducing nitrobenzenesulfo compounds" Seite 470, Spalte 1, Zusammenfassung Nr. 29 367 w**

(73) Patentinhaber : **BASF Aktiengesellschaft**
**Carl-Bosch-Strasse 38**
**D-6700 Ludwigshafen (DE)**

(72) Erfinder : **Hackenberger, Alfred, Dr. Chem.**
**Luitpoldstrasse 77**
**D-6700 Ludwigshafen (DE)**
Erfinder : **Patsch, Manfred, Dr. Chem.**
**Fritz-Wendel-Strasse 4**
**D-6706 Wachenheim (DE)**

**EP 0 146 889 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von aromatischen Aminosulfonsäuren durch Hydrierung von aromatischen Nitrosulfonsäuren in Gegenwart von Raney-Nickel und/oder Raney-Cobalt, und

a) 1 bis 25 Gew.% Borsäure und/oder Borat und

b) 0,1 bis 0,9 Gew.% Phosphorsäure und/oder Phosphat,

bezogen auf Ausgangsstoff II, bei einem Druck zwischen 1 und 5 bar.

Aminobenzosulfonsäuren sind wichtige Ausgangsprodukte insbesondere für die Herstellung von Farbstoffen. Sie werden im allgemeinen durch Béchamp-Reduktion der entsprechenden Nitrobenzolsulfonsäuren bzw. deren Salze erhalten. Dieses Verfahren besitzt jedoch eine Reihe von Nachteilen : die Abtrennung des Endstoffs vom Eisenoxidschlamm ist zeitaufwendig und führt zu Ausbeuteminderungen durch Anteile, die im Eisenoxidschlamm verbleiben. Außerdem fallen größere Mengen an Natriumacetat zwangsmäßig an, da im allgemeinen in essigsaurer Lösung bei einem pH-Wert von 3 gearbeitet wird.

Versuche, die Béchamp-Reduktion durch die katalytische Hydrierung mit Raney-Nickel als Katalysator zu ersetzten, hatten häufig nicht den gewünschten Erfolg : die Wasserstoffaufnahme erfolgt nicht quantitativ, da der Katalysator durch schwefelhaltige Verbindungen vergiftet wird. Um einen vollständigen Umsatz zu erreichen, muß bei höheren Drücken (20-200 bar) gearbeitet werden, was den Einsatz teuerer Hochdruckapparate erforderlich macht. Bei niederen Drücken kann eine vollständige Hydrierung von Nitrobenzolsulfonsäuren nur in Gegenwart teurer Palladium- oder Platinkatalysatoren erfolgen.

Aus diesen Gründen wurden schon verschiedene Säuren bzw. deren Salze als Promotoren für das wirtschaftlich günstige Raney-Nickel als Katalysator bei der Hydrierung von Nitrobenzolsulfonsäuren in wäßriger Lösung geprüft. In der DE-OS 23 01 739 werden Borsäure bzw. Borsäure abgebende Mittel als Promotoren bei der katalytischen Hydrierung von m-Nitrobenzolsulfonsäuren in Gegenwart von Raney-Nickel bei einem Druck von 1-5 Atmosphären vorgeschlagen. Wie jedoch Vergleichsbeispiel 3 zeigt, werden dabei lange Reaktionszeiten benötigt. Das Verfahren wird deshalb nicht im großtechnischen Maßstab durchgeführt, da sich die Reaktionsbeschleunigung durch die Borsäure als unzureichend erweist.

Es wird in CA *78* (1973), 29367w beschrieben, daß die katalytische Aktivität von mit Palladium dotiertem Raney-Nickel bei der Reduktion von Salzen von Nitrobenzolsulfonsäuren in wäßrigem Medium durch Zugabe von $NaH_2PO_4$ erhöht wird.

Wie J. of Applied Chemistry UdSSR, Vol 45 (1972), 2825-2828 zeigt, wird auch die Aktivität von undotiertem Raney-Nickel bei der entsprechenden katalytischen Hydrierung durch Zugabe von Phosphat bzw. Sulfat erhöht. Unter diesen Bedingungen kann die Reduktion von Natrium-m-nitrobenzolsulfonat bei 25 °C und drucklos durchgeführt werden. Es muß jedoch mit einer derart hohen Menge an Raney-Nickel gearbeitet (Gewichtsverhältnis Nitroverbindung : Raney-Nickel = 3 : 5) werden, daß dem Verfahren keine wirtschaftliche Bedeutung zukommt. Die Veröffentlichung hebt als empfehlenswertes Katalysatorsystem Raney-Nickel mit Palladium als Promotor und einem Zusatz von Mononatriumphosphat hervor.

Die DE-OS 22 40 849 beschreibt die Schwierigkeit der katalytischen Hydrierung von Nitrobenzolen, die durch Halogenatome, Halogenalkylgruppen und aliphatischen Sulfonylgruppen substituiert sind, da solche Stoffe entweder nicht hydriert werden oder bei der Hydrierung in erheblichem Maße zu Nebenreaktionen neigen. Für die Hydrierung von so substituierten Ausgangsstoffen werden ein Zusatz von Sauerstoffsäuren des drei- oder fünfwertigen Phosphors, des Bors, der Kohlensäure oder niedermolekularer Carbonsäuren und ausreichend starker Basen und eine Arbeitsweise mit einem pH-Wert von 6 bis 7,5 angegeben. Wie alle Beispiele zeigen, muß mit einem Druck von 40 bar umgesetzt werden. Nachteilig ist die zahlreiche Arbeitsschritte umfassende Aufarbeitung (Beispiel 1) : Das Reaktionsgemisch wird auf 95 °C erhitzt und mit Natronlauge auf pH 10,7 gestellt und dann werden Aktivkohle und Kieselgur zugegeben. Anschließend wird das Gemisch erneut gerührt und bei 95 °C über eine beheizte Drucknutsche geklärt, dann das Filtrat mit Salzsäure auf einen pH-Wert von 5,2 gestellt, die so gebildete Suspension auf 0 °C gekühlt und filtriert. Das Filtergut wird mit Natriumhydrogensulfitlösung behandelt und erneut abgesaugt, dann wird dieser Reinigungsvorgang viermal durchgeführt. Wie die Beispiele zeigen, wird stets nur mit einem Promotor (Säure) umgesetzt. Die Hydrierung einer Chlornitrobenzolsulfonsäure (Beispiel 7) wird bei 40 bar und mit einem Zusatz von Dinatriumhydrogenphosphit und wäßriger Natriumhydroxidlösung durchgeführt. Im Zusammenhang mit Nitrosulfonsäuren als Ausgangsstoffen werden weder Phosphorsäure noch Borsäure bzw. ihre Salze aufgeführt.

In der DE-OS 30 03 254 werden Ester der Sauerstoffsäuren des drei- oder fünftwertigen Phosphors oder eine neutralisierte wäßrige Lösung dieser Säuren selbst als Promotoren für die katalytische Hydrierung von organischen Verbindungen genannt. Die Hydrierung wird in Gegenwart von etwa 1-10 Gew.% des Promotors, bezogen auf die zu hydrierende Ausgangsverbindung, durchgeführt. Es muß, wie alle Beispiele zeigen, mit Drücken von 40 bar und mehr hydriert werden.

Aromatische Nitroverbindungen werden in den Beispielen nicht gezeigt. Lediglich 2-Nitropropan (Beispiel 11) wird bei 40 bar Druck und unter Verwendung einer mit Natronlauge neutralisierten Natriumdihydrogenphosphatlösung als Promotor hydriert.

Alle diese Verfahren befriedigen gerade im großtechnischen Maßstab mit Bezug auf einfachen und wirtschaftlichen Betrieb, Verwendung niedriger Drücke, Ausbeute sowie Raum-Zeit-Ausbeute nicht.

Es wurde nun gefunden, daß man aromatische Aminosulfonsäuren der Formel I,

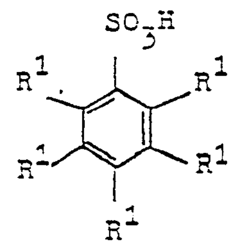

(I)

worin die einzelnen Reste $R^1$ gleich oder verschieden sein können, mindestens ein Rest $R^1$ eine Aminogruppe bezeichnet, die übrigen Reste $R^1$ jeweils einen aliphatischen, araliphtischen, aromatischen Rest, ein Wasserstoffatom, eine Hydroxygruppe, eine Sulfonsäuregruppe oder den Rest

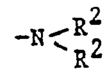

bedeuten, die einzelnen Reste $R^2$ gleich oder verschieden sein können und jeweils für einen aliphatischen, araliphatischen oder aromatischen Rest stehen, ein Rest $R^2$ auch ein Wasserstoffatom bezeichnen kann, jeweils zwei benachbarte Reste $R^1$ zusammen mit den beiden benachbarten Kohlenstoffatomen auch Glieder eines anellierten aromatischen Restes bezeichnen können, durch Hydrierung von aromatischen Nitrosulfonsäuren in Gegenwart von Hydrierkatalysatoren und Säuren oder Salzen unter Druck vorteilhaft erhält, wenn man aromatische Nitrosulfonsäuren der Formel II,

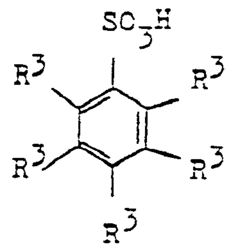

(II)

worin die einzelnen Reste $R^3$ gleich oder verschieden sein können, mindestens ein Rest $R^3$ eine Nitrogruppe bedeutet, die übrigen Reste $R^3$ die Bedeutung von $R^1$ besitzen, bei einem Druck zwischen 1 und 5 bar in Gegenwart von Raney-Nickel und/oder Raney-Cobalt und

a) 1 bis 25 Gew.% Borsäure und/oder Borat und

b) 0,1 bis 0,9 Gew.% Phosphorsäure und/oder Phosphat,

bezogen auf Ausgangsstoff II, hydriert.

Die Umsetzung kann für den Fall der Verwendung von 4-Nitrodiphenylamin-2-sulfonsäure durch die folgenden Formeln wiedergegeben werden :

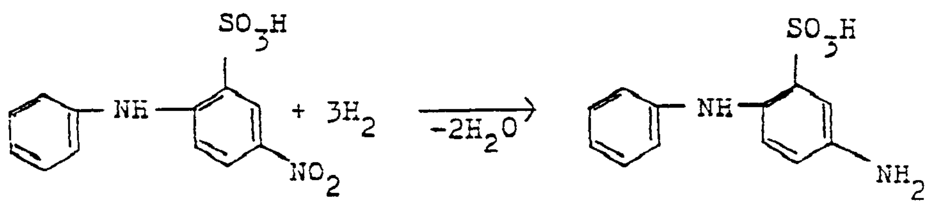


Im Hinblick auf die bekannten Verfahren liefert das Verfahren nach der Erfindung auf einfacherem und wirtschaftlicherem Wege aromatische Aminosulfonsäuren in besserer Ausbeute und Reinheit. Die Aminosulfonsäuren I können so weiterverwendet werden, wie sie bei der erfindungsgemäßen Herstellung anfallen, ein zusätzlicher Reinigungsschritt ist nicht erforderlich. Alle diese vorteilhaften Ergebnisse sind mit Bezug auf den Stand der Technik überraschend. Es konnte gerade im Hinblick auf die DE-OS 22 40 849 und DE-OS 30 03 254 nicht erwartet werden, daß eine Kombination der erfindungsgemäßen Promotoren und dazu noch in den erfindungsgemäßen Mengenverhältnissen und bei dem erfindungsgemäßen Druck erheblich bessere Ergebnisse ergibt.

Bevorzugte Ausgangsstoffe II und dementsprechend bevorzugte Endstoffe I sind solche, in deren Formeln die einzelnen Reste $R^1$ gleich oder verschieden sein können, mindestens ein Rest $R^1$, zweckmäßig 1 bis 3, vorteilhaft 1 oder 2 Reste $R^1$ jeweils eine Aminogruppe bezeichnen, die übrigen Reste $R^1$ jeweils einen Alkylrest von 1 bis 8, insbesondere 1 bis 4 Kohlenstoffatomen, einen Aralkylrest

oder Alkylarylrest mit 7 bis 12 Kohlenstoffatomen, einen Phenylrest, ein Wasserstoffatom, eine Hydroxygruppe, eine Sulfonsäuregruppe oder den Rest

$$-N\begin{matrix} R^2 \\ R^2 \end{matrix}$$

bedeuten, die einzelnen Reste $R^2$ gleich oder verschieden sein können und jeweils für einen Alkylrest mit 1-8, insbesondere 1-4 Kohlenstoffatomen, einen Aralkylrest oder Alkylarylrest mit 7-12 Kohlenstoffatomen, einen Phenylrest stehen, ein Rest $R^2$ auch ein Wasserstoffatom bezeichnen kann, jeweils zwei benachbarte Reste $R^1$ zusammen mit den beiden benachbarten Kohlenstoffatomen auch Glieder eines anellierten Phenylenrestes bezeichnen können, die einzelnen Reste $R^3$ gleich oder verschieden sein können, mindestens 1 Rest $R^3$, zweckmäßig 1-3, vorteilhaft 1 oder 2 Reste $R^3$ jeweils eine Nitrogruppe bedeuten, die übrigen Reste $R^3$ die Bedeutung von $R^1$ besitzen. Die vorgenannten Reste können noch durch unter den Reaktionsbedingungen inerte Gruppen, z. B. Alkylgruppen mit 1 bis 4 Kohlenstoffatomen, substituiert sein. Die Aminogruppen der Endstoffe I werden durch Hydrierung der Nitrogruppen der Ausgangsstoffe II erhalten.

Beispielsweise sind folgende Ausgangsstoffe II geeignet : ortho-, meta-, para-Nitrobenzolsulfonsäure ; entsprechende in 2-, 3-, 4-, 5- oder 6-Stellung einfach oder in 2,3-, 2,4-, 2,5-, 2,6-, 3,4-, 3,5-Stellung gleich oder verschieden zweifach durch die Methyl-, Ethyl-, Propyl-, Isopropyl-, Butyl-, sec. Butyl-, Isobutyl-, tert. Butyl-, Benzyl-, Phenyl-, Hydroxi-, Sulfonsäure-, Methylamino-, Ethylamino-, Propylamino-, Isopropylamino-, Butylamino-, Isobutylamino-, sec. Butylamino-, tert. Butylamino-, Benzylamino-, Phenylamino-, Dimethylamino-, Diethylamino-, Dipropylamino-, Diisopropylamino-, Dibutylamino-, Disobutylamino-, Di-sec.-Butylamino-, Di-tert.-Butylamino-, Dibenzylamino-, Diphenylamino-Gruppen substituierte Nitrobenzolsulfonsäuren ; analoge unsubstituierte oder in vorgenannter Weise substituierte Dinitrobenzolsulfonsäuren, deren Nitrogruppen in ortho-, meta- oder para-Stellung zueinander stehen ; bevorzugt 4-Nitrodiphenylamin-2-sulfonsäure und Dinitromesitylensulfonsäure.

Die Umsetzung wird in der Regel bei einer Temperatur von 20 bis 100 °C, vorzugsweise von 50 bis 95 °C, kontinuierlich oder diskontinuierlich durchgeführt. Drücke zwischen 1 und 5 bar, vorteilhaft von 2 bis 4 bar werden verwendet. Zweckmäßig kommt als Lösungsmittel Wasser, im allgemeinen in Abwesenheit zusätzlicher organischer Lösungsmittel, in Betracht. Vorteilhaft sind im Ausgangsgemisch insgesamt Mengen von 5 bis 40, bevorzugt 10 bis 30 Gew.% Ausgangsstoff II, bezogen auf die eingesetzte Menge Wasser enthalten. Das Wasser kann als getrennter Zusatz oder zusammen mit anderen Stoffen, z. B. in Gestalt wäßriger Lösungen von Phosphorsäure oder Natriumhydroxid, dem Ausgangsgemisch zugeführt werden. Man verwendet bei der Hydrierung zweckmäßig pH-Werte von 4 bis 9, bevorzugt 6 bis 7,6.

Die Umsetzung wird mit Wasserstoff in stöchiometrischer Menge oder im Überschuß, zweckmäßig in einer Menge von 3 bis 3,2, vorzugsweise von 3 bis 3,05 Mol $H_2$ je Mol Ausgangsstoff II und Nitrogruppe, durchgeführt. Man kann den Wasserstoff kontinuierlich oder diskontinuierlich der Reaktion zuführen und/oder den Katalysator selbst nach einer bestimmten Reaktionszeit wieder frisch mit Wasserstoff beladen. Als Katalysatoren dienen Raney-Kobalt und/oder bevorzugt Raney-Nickel, zweckmäßig in einer Menge von 0,1 bis 20, bevorzugt 1 bis 10 Gew.%, bezogen auf Ausgangsstoff II, und als Promotoren ein Gemisch von Borsäure und Phosphorsäure. Diese Säuren können ganz oder teilweise durch ihre Salze, bevorzugt Alkalisalze, insbesondere Natrium- und Kalisalze, ersetzt werden. Als Phosphate kommen Mono-, Di- und Trialkaliphosphate, bevorzugt Monoalkalidihydrogenphosphate in Betracht. Man verwendet Mengen von 1 bis 25, vorzugsweise 5 bis 20 Gew.% Borsäure und/oder 1 bis 25, bevorzugt 5 bis 20 Gew.% Borat, und 0,1 bis 0,9, bevorzugt 0,4 bis 0,8 Gew.% Phosphorsäure und/oder 0,1 bis 0,9, bevorzugt 0,4 bis 0,8 Gew.% Phosphat, bezogen auf Ausgangsstoff II. Es können Gemische A) der beiden Säuren oder B) der Salze oder C) beider Säuren und beider Salze oder D) eines Salzes und beider Säuren oder E) einer Säure und beider Salze verwendet werden, zweckmäßig insgesamt 1 bis 80, bevorzugt 15 bis 50 Mol Borsäure (als freie Säure und/oder als Salz) je Mol Phosphorsäure (freie Säure und/oder in Gestalt des Salzes). Zweckmäßig werden dem Ausgangsgemisch solche Mengen an Basen, vorteilhaft Alkalihydroxyde, insbesondere Natronlauge, zugesetzt, daß sich die vorgenannten pH-Werte einstellen.

Die Reaktion kann wie folgt durchgeführt werden : Ein Gemisch von Ausgangsstoff II, Wasser, Promotoren, Wasserstoff, Hydrierkatalysator und gegebenenfalls Base zur Einstellung des pH-Wertes wird zweckmäßig während 1 bis 20 Stunden, vorzugsweise von 2 bis 10 Stunden bei der Reaktionstemperatur und dem vorgenannten Reaktionsdruck gehalten. Man gibt zweckmäßig in einem Reaktor einem Gemisch von Wasser und Ausgangsstoff II die Phosphorsäure bzw. das Phosphat oder beide Promotoren zu, stellt mit der Base, z. B. NaOH, den vorgenannten pH-Wert ein, setzt den Hydrierkatalysator und gegebenenfalls Borsäure, Borat oder einen Anteil beider Promotoren zu und spült den Reaktionsraum mit Stickstoff. Dann wird Wasserstoff bis zu vorgenanntem Reaktionsdruck eingepreßt. Nun wird das Reaktionsgemisch auf vorgenannte Temperatur gebracht und solange bei dieser Temperatur unter Einleitung von weiterem Wasserstoff gehalten, bis kein Wasserstoff mehr durch die Reaktion verbraucht wird. Nun wird das Reaktionsgemisch abgekühlt und filtriert. Aus dem Filtrat wird der Endstoff nach den üblichen Methoden, z. B. durch Ansäuern des Filtrats und Filtration, abgetrennt.

Die nach dem Verfahren der Erfindung herstellbaren Verbindungen sind wertvolle Zwischenprodukte für die Herstellung von Farbstoffen, Herbiziden, Fungiziden und Pharmazeutika. Bezüglich der Verwendung wird auf die vorgenannten Veröffentlichungen und Ullmanns Encyklopädie der Technischen Chemie (4. Aufl.), Band 8, Seiten 425-429, verwiesen.

Beispiel 1

4,64 Kilogramm Dinitromesitylensulfonsäure wurden in einem 50-1-Rührkessel in 18 Kilogramm Wasser suspendiert. Die Suspension wurde mit 0,036 Kilogramm 85 gew.%iger Phosphorsäure versetzt und mit 0,4 Kilogramm 50 gew.%iger Natronlauge auf einen pH-Wert von 7,5 eingestellt. Nach Zugabe von 0,72 Kilogramm Borsäure und 0,18 Kilogramm Raney-Nickel wurde der Kessel verschlossen, zweimal mit Stickstoff und einmal mit Wasserstoff gespült und anschließend Wasserstoff aufgebreßt, so daß der Druck 3 bar betrug. Man erwärmte auf 90 °C und hydrierte bei dieser Temperatur und einem Druck von 3 bar unter kräftigem Rühren. Nach insgesamt 4 Stunden Reaktionszeit war die Wasserstoffaufnahme beendet ; es wurden insgesamt 590 l Wasserstoff (3 bar) aufgenommen. Man kühlte die Lösung auf 60 °C und heberte sie über ein Seitz-Filter ab. Man stellte die Lösung bei 50 °C mit 1,8 Kilogramm HCl konz. auf pH 1-2 und nutschte den Niederschlag ab. Es wurden nach Neutralwaschen 3,46 Kilogramm Diaminomesitylensulfonsäure von einer Reinheit von 95 % erhalten. Dies entsprach einer Ausbeute von 94 % der Theorie. Zersetzungstemperatur des Endstoffs oberhalb von 318 °C.

Beispiel 2 und Vergleichsbeispiele 3-5

Analog Beispiel 1 wurden die folgenden Hydrierungen mit unterschiedlichen Promotorenmengen durchgeführt.

| Beispiel | Promotor | Hydrierzeit | Ausbeute in % der Theorie |
|---|---|---|---|
| 2 | 0,72 Kilogramm Borsäure + 0,072 Kilogramm $H_3PO_4$ (85 Gew.%) | 4 Stunden | 94 |
| 3 (Vergleich) | 0,72 Kilogramm Borsäure | 10 Stunden | 90 |
| 4 (Vergleich) | 0,072 Kilogramm $H_3PO_4$ (85 Gew.%) | 30 Stunden | $< 10$ |
| 5 (Vergleich) | - | 30 Stunden | $< 16$ |

Beispiel 6

In einer 50-1-Hydrierapparatur wurden 4,4 Kilogramm 4-Nitrodiphenylamin-2-sulfonsäure in 25 l Wasser gelöst und mit 0,345 Kilogramm Borsäure sowie 0,03 Kilogramm 85 gew.%iger Phosphorsäure versetzt. Der pH-Wert der Lösung wurde mit konz. Salzsäure auf 6,5 eingestellt. Danach wurden 0,2 Kilogramm Raney-Nickel zugegeben, der Kessel verschlossen und, wie in Beispiel 1 beschrieben, hydriert und aufgearbeitet (Hydrierzeit 6 Stunden). Es wurden 3,26 Kilogramm 4-Aminodiphenylamin-2-sulfonsäure mit einer Zersetzungstemperatur > 200 °C erhalten. Dies entspricht einer Ausbeute von 82,5 % der Theorie.

**Patentanspruch**

Verfahren zur Herstellung von aromatischen Aminosulfonsäuren der Formel I

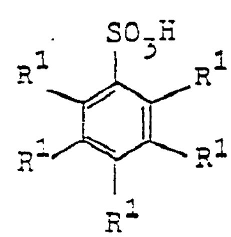

(I)

worin die einzelnen Reste $R^1$ gleich oder verschieden sein können, mindestens ein Rest $R^1$ eine Aminogruppe bezeichnet, die übrigen Reste $R^1$ jeweils einen aliphatischen, araliphatischen, aromatischen Rest, ein Wasserstoffatom, eine Hydroxygruppe, eine Sulfonsäuregruppe oder den Rest

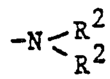

bedeuten, die einzelnen Reste $R^2$ gleich oder verschieden sein können und jeweils für einen aliphatischen, araliphatischen oder aromatischen Rest stehen, ein Rest $R^2$ auch ein Wasserstoffatom bezeichnen kann, jeweils zwei benachbarte Reste $R^1$ zusammen mit den beiden benachbarten Kohlenstoffatomen auch Glieder eines anellierten aromatischen Restes bezeichnen können, durch Hydrierung von aromatischen Nitrosulfonsäuren in Gegenwart von Hydrierkatalysatoren und Säuren oder Salzen unter Druck, dadurch gekennzeichnet, daß man aromatische Nitrosulfonsäuren der Formel II,

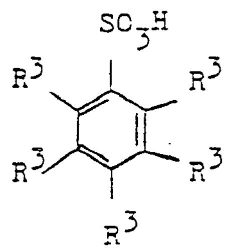

(II)

worin die einzelnen Reste $R^3$ gleich oder verschieden sein können, mindestens ein Rest $R^3$ eine Nitrogruppe bedeutet, die übrigen Reste $R^3$ die Bedeutung von $R^1$ besitzen, bei einem Druck zwischen 1 und 5 bar in Gegenwart von Raney-Nickel und/oder Raney-Cobalt und

a) 1 bis 25 Gew.% Borsäure und/oder Borat und

b) 0,1 bis 0,9 Gew.% Phosphorsäure und/oder Phosphat, bezogen auf Ausgangsstoff II, hydriert.

**Claim**

A process for the preparation of an aromatic aminosulfonic acid of the formula I

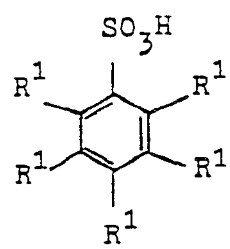

(I)

where the individual radicals $R^1$ can be identical or different, at least one radical $R^1$ is amino and the remaining ones are each an aliphatic, araliphatic or aromatic radical, hydrogen, hydroxyl, sulfo or a radical

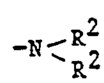

and individual radicals $R^2$ can be identical or different and are each an aliphatic, araliphatic or aromatic radical, and one radical $R^2$ may furthermore be hydrogen, and two adjacent radicals $R^1$, together with the two adjacent carbon atoms, may furthermore be members of a fused aromatic radical, by hydrogenating an aromatic nitrosulfonic acid in the presence of a hydrogenation catalyst and acids or salts under superatmospheric pressure, wherein an aromatic nitrosulfonic acid of the formula II

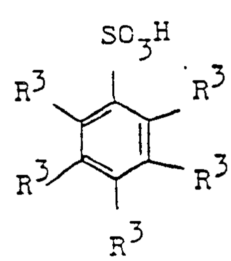

(II)

where the individual radicals $R^3$ can be identical or different, at least one radical $R^3$ is nitro and the remaining ones have the same meanings as $R^1$, is hydrogenated under a pressure of between 1 and 5 bar, in the presence of Raney nickel and/or Raney cobalt and

a) from 1 to 25 % by weight of boric acid and/or a borate, and

b) from 0.1 to 0.9 % by weight of phosphoric acid and/or a phosphate, the percentages being based on starting material II.

**Revendication**

Procédé de fabrication d'acides aminosulfoniques aromatiques de formule I

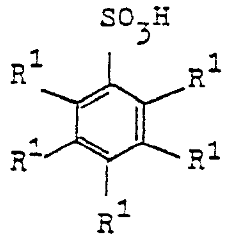

(I)

dans laquelle les restes individuels $R^1$ peuvent être identiques ou différents, au moins un résidu $R^1$ désignant un groupe amino, les autres restes $R^1$ représentant chaque fois un reste aliphatique, araliphatique, aromatique, un atome d'hydrogène, un groupe hydroxy, un groupe acide sulfonique ou bien le reste

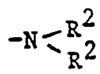

dans lequel les restes individuels $R^2$ peuvent être identiques ou différents, et sont chaque fois mis pour un reste aliphatique, araliphatique ou aromatique, un reste $R^2$ pouvant même désigner un atome d'hydrogène, deux restes voisins $R^1$, conjointement avec les deux atomes de carbone voisins, pouvant chaque fois désigner également des éléments d'un reste aromatique condensé, par hydrogénation d'acides nitrosulfoniques aromatiques en présence de catalyseurs d'hydrogénation et d'acides ou de sels sous pression, caractérisé par le fait que l'on hydrogène des acides nitrosulfoniques aromatiques de la formule II,

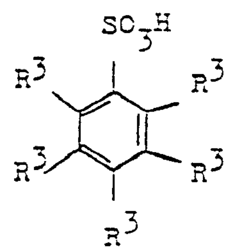

(II)

dans laquelle les restes individuels $R^2$ peuvent être identiques ou différents, un reste $R^3$ au moins désigne un groupe nitro, les autres restes $R^3$ possédant la signification de $R^1$, et ce à une pression comprise entre 1 et 5 bars, en présence de nickel de Raney et/ou de cobalt de Raney et de :

a) 1 à 25 % en poids d'acide borique et/ou de borate, et

b) 0,1 à 0,9 % en poids d'acide phosphorique et/ou de phosphate, rapporté au produit de base II.